# EUROPEAN PATENT SPECIFICATION

(11) **EP 2 323 718 B1**
(45) Date of publication and mention of the grant of the patent: **09.10.2019**
(21) Application number: 08789753.4
(22) Date of filing: 06.08.2008
(51) Int. Cl.: A61M 5/20, A61M 5/48, A61M 5/30

(54) **INTRADERMAL NEEDLE-LESS INJECTION MECHANISM**
INTRADERMALER NADELLOSER INJEKTIONSMECHANISMUS
MÉCANISME D'INJECTION INTRADERMIQUE SANS AIGUILLE

(43) Date of publication of application: 25.05.2011
(73) Proprietor: Perf-Action Technologies Ltd., 76326 Rehovot (IL)
(72) Inventor: BAROR, Eyal, 60850 Shoham (IL); LEV KONYA, Gad, 79850 Neve Mivtach (IL)
(74) Representative: Croston, David
(86) International application number: PCT/IL2008/001077
(87) International publication number: WO 2010/016049

(56) References cited:
- WO-A1-02/060516
- US-A- 2 754 818
- US-A1- 2004 254 526
- US-A1- 2005 192 530
- US-A1- 2007 191 762
- US-A1- 2008 009 788
- US-A1- 2008 086 079

## Description

### FIELD OF THE INVENTION

The present invention relates generally to needle-less injection of fluids subcutaneously or intradermaly, and specifically to methods and mechanisms for performing such.

### BACKGROUND OF THE INVENTION

In many medical and aesthetic procedures there is a requirement to transfer fluids to areas below the surface of the skin, for example injecting insulin to diabetics and in aesthetic applications filling the dermal layer of the skin with collagens or other materials to preserve a youthful look. In PCT application number PCT/IL2007/000796 filed on August 3, 2007 there is described an intradermal needle-less injection device for injecting fluids into the skin below the epidermis layer. Other devices are also known in the art.

When injecting fluid with a needle-less injection device the fluid needs to arrive at the epidermis at a high pressure so that the fluid will penetrate it and enter the skin. If the fluid hits the epidermis at a low pressure it will disperse on the surface of the skin and not be delivered subcutaneously. Typically a pressure that exceeds 100 Atmospheres, for example between 120 - 150 Atmospheres is required to penetrate the epidermis.

Another problem is to control the dispersion of the fluid in the dermal layer. In some aesthetic applications it is desirable that the fluid spread out evenly over a wide area under the epidermis. Sometimes the spreading contributes to traumatizing the dermal layer near the epidermis, and provoking a wound healing process, which initiates cell rejuvenation. However, a fluid being administered at high pressures will tend to penetrate substantially in a strait line into the dermal layer and continue into the subcutaneous and even muscle layers and not spread out perpendicular (e.g. laterally) to the direction of penetration.

Therefore, a need exists for performing a needle-less delivery of liquid into the skin layers, whereas the delivery will disperse more laterally inside the skin, after penetrating the outermost layer.

US patent publication 2005/192530 to Castellano describes various apparatuses and methods for administering a needle-less injection of a degassed fluid.

### SUMMARY OF THE INVENTION

An aspect of an embodiment of the invention, relates to a method and mechanism for performing needle-less injection of a dose of fluid through the epidermis into the dermal layer. Initially a high injection pressure is applied momentarily to penetrate the epidermis and prevent dispersion of the fluid on the surface of the skin. After forming an initial penetration hole the pressure is reduced for the rest of the flow of the fluid dose through the penetration hole to enhance dispersion in the dermal layer.

In an exemplary embodiment of the invention, the dose of fluid is injected as a limited continuous flow by being pushed out of a nozzle head using a plunger. The initial pressure is formed by using compressed gas to thrust a hammer head against the plunger, wherein the initial impact of the hammer head, by its kinetic energy, causes the plunger to push fluid from the nozzle at a high pressure toward the surface of the skin of a patient. Following the initial impact a steady force of a lower pressure is exerted on the hammer head. The lower pressure is high enough to continue pushing the hammer head. The hammer head pushes the plunger to empty the rest of the content of the nozzle through the penetration hole into the dermal layer of the patient's skin.

There is thus provided according to an exemplary embodiment of the invention, a needle-less fluid injection mechanism, comprising:
a nozzle for accepting a dose of fluid to be injected into the skin of a patient;
a plunger that is adapted to be pushed into the nozzle to inject the dose of fluid from the nozzle;
a hammer that is adapted to be thrust at and to collide with the plunger to cause the release of a first amount of fluid from the nozzle at a high pressure and then to release the rest of the fluid from the nozzle by continuing to push the plunger at a lower pressure until injecting the entire dose of fluid, characterized by using compressed air to thrust said hammer toward said plunger and to continue pushing said plunger with said hammer. In an exemplary embodiment of the invention, the mechanism further comprises a two plated piston which separates between 3 air chambers. Optionally, the release of air pressure from the uppermost chamber causes the two plated piston to move and release the air pressure from the middle chamber toward the hammer. In the invention, the mechanism further comprises a positioning motor to position the plunger before thrusting the hammer. Optionally, the positioning motor is adapted to form a gap between the hammer and the plunger before thrusting the hammer toward the plunger. In an exemplary embodiment of the invention, the level of the pressure by which the first amount of fluid is released is controlled by adjusting the pressure of the compressed air used. Alternatively or additionally, the level of the pressure by which the first amount of fluid is released is controlled by adjusting the impact parameters of the hammer.

### BRIEF DESCRIPTION OF THE DRAWINGS

The present invention will be understood and better appreciated from the following detailed description taken in conjunction with the drawings. Identical structures, elements or parts, which appear in more than one figure, are generally labeled with the same or similar number in all the figures in which they appear, wherein:
Fig. 1A is a schematic illustration of a needle-less injection mechanism in a first position, according to an exemplary embodiment of the invention;
Fig. 1B is a schematic illustration of a needle-less injection mechanism in a second position, according to an exemplary embodiment of the invention;
Fig. 1C is a schematic illustration of a needle-less injection mechanism in a third position, according to an exemplary embodiment of the invention;
Fig. 2 is a flow diagram of the process of injecting fluid using a needle-less injection mechanism, according to an exemplary embodiment of the invention;
Fig. 3 is an illustration of schematic graphs depicting pressure versus time for a needle-less injection mechanism with a hammer head relative to a mechanism without a hammer head, according to an exemplary embodiment of the invention; and
Fig. 4 is an illustration of the dispersion of the injected fluid into the skin using a needle-less injection device with a hammer head relative to a mechanism without a hammer head, according to an exemplary embodiment of the invention.

### DETAILED DESCRIPTION

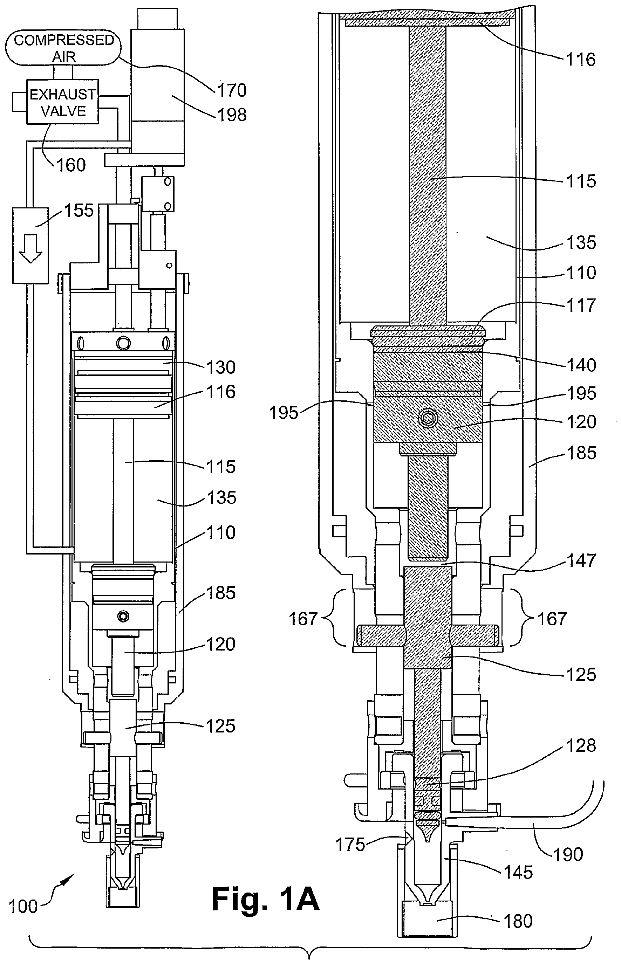

Figures 1A to 1C are schematic illustrations of a needle-less injection mechanism 100 in a first, second and third position, according to an exemplary embodiment of the invention. In an exemplary embodiment of the invention, mechanism 100 comprises an encasement 110 that encloses a two plated piston 115 (including upper plate 116, lower plate 117 and a connecting shaft), a hammer head 120, and a plunger 125. Optionally, piston 115 separates 3 air chambers (130, 135 and 140) in encasement 110, which will be manipulated to release air at a high pressure to push hammer head 120 onto plunger 125. In an exemplary embodiment of the invention, hammer head 120 is designed to be thrust forward by compressed air 170 to collide with plunger 125 and transfer its momentum to plunger 125. Plunger 125 is coupled to an injection head 128 that pushes a fluid 165 that was pre-loaded into a nozzle 145 toward the skin of a patient. In an exemplary embodiment of the invention the compressed air 170 will continue to push hammer head 120 onto plunger 125 until the fluid content in nozzle 145 will be pushed out toward the patient. The initial collision will cause fluid to be released at a higher pressure than the pressure exerted afterwards. At the same time compressed air 170 in encasement 110 is expanding and pushes hammer head 120 forward after the collision.

In some embodiments of the invention, mechanism 100 is made from metal, for example stainless steel, aluminum or any other metals or combination of metals. Alternatively or additionally, mechanism 100 may include plastics, rubber and/or any other material known in the art with or without metal parts. In some embodiments of the invention, a specific compressed gas may be used instead of compressed air 170.

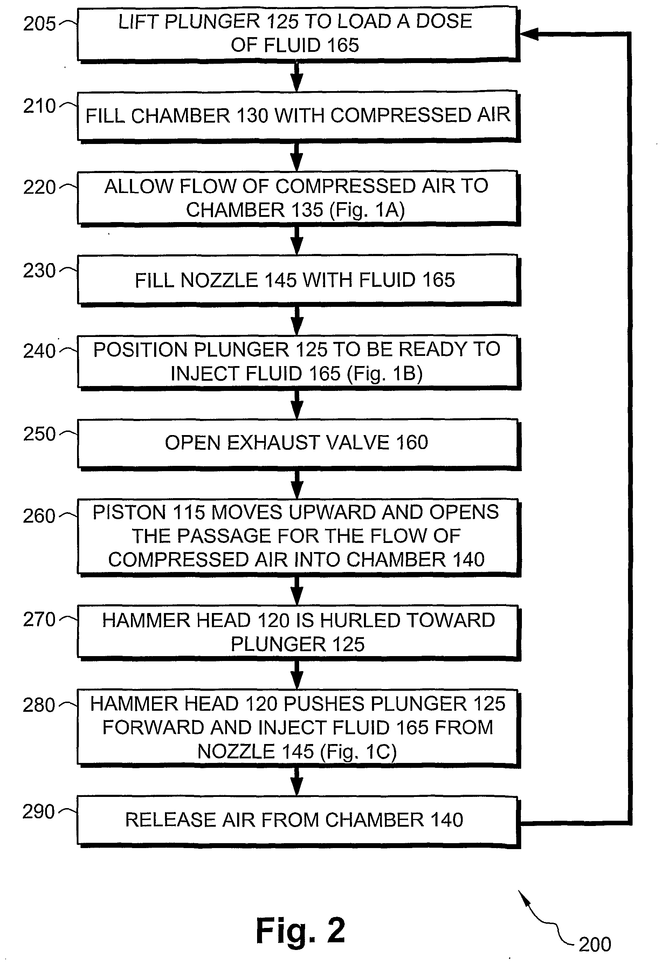

Fig. 2 is a flow diagram 200 of the process of injecting fluid using needle-less injection mechanism 100, according to an exemplary embodiment of the invention. Positioning motor 198 controls a positioning mechanism 185 that connects between positioning motor 198 and plunger 125. Positioning mechanism 185 is used to lift plunger 125 (205) upward so that nozzle 145 will be ready to be loaded with a dose of fluid 165. Additionally, by lifting plunger 125 also hammer head 120 is lifted and the volume of chamber 140 is reduced or canceled. In some embodiments of the invention, positioning motor 198 is an electronic motor. Alternatively, positioning motor 198 may be a pneumatically controlled motor.

In an exemplary embodiment of the invention, compressed air 170 is provided to fill (210) chamber 130. By filling chamber 130, piston 115 is pushed downward so that it seals between chamber 135 and chamber 140. In an exemplary embodiment of the invention, chamber 135 is situated between the two plates of piston 115, wherein the upper plate 116 separates between chamber 130 and chamber 135, and the lower plate 117 separates between chamber 135 and chamber 140 when piston 115 is pushed down. A one way flow control 155 allows the flow (220) of the compressed air 170 that is provided to chamber 130 also to chamber 135, so that chambers 130 and 135 will have the same pressure. In an exemplary embodiment of the invention, nozzle 145 is then filled (230) with a dose of fluid 165 for injecting onto a patient, via a tube 190.

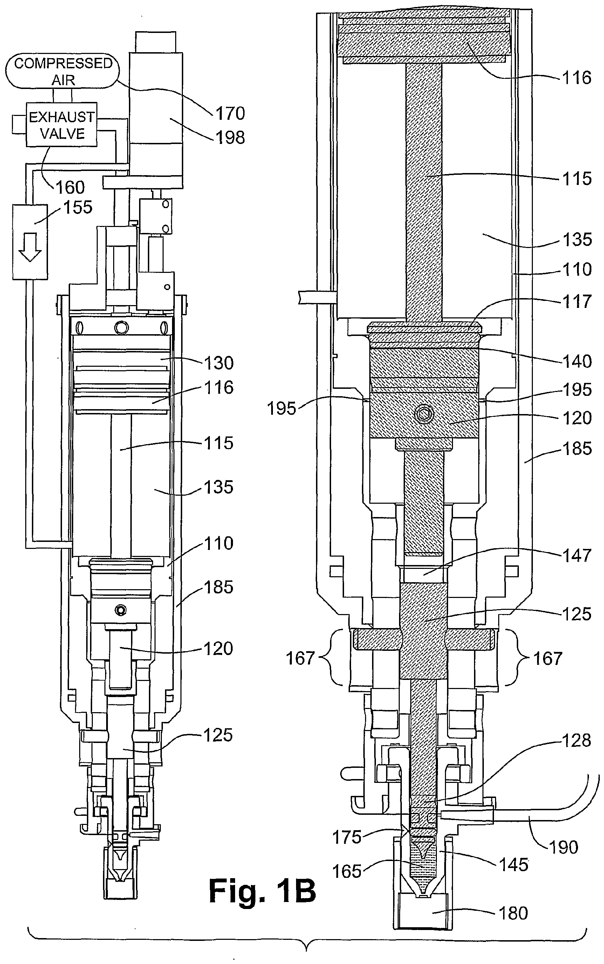

In an exemplary embodiment of the invention, after loading a dose of fluid 165, positioning mechanism 185 grasps plunger 125 and positions (240) injection head 128 that is coupled to plunger 125 in contact with fluid 165 as shown in Fig. 1B. The positioning of plunger 125 releases trapped air through one or more vents 175, so that fluid 165 is isolated and ready for injection. Additionally, the positioning of plunger 125 leaves a gap 147 between plunger 125 and hammer head 120. In an exemplary embodiment of the invention, positioning mechanism 185 is adapted to pull and push plunger 125 with a slight offset 167 to allow plunger 125 and injection head 128 to be initially pulled up above tube 190 and vent 175, and then pushed down onto fluid 165 leaving gap 147 between plunger 125 and hammer head 120. Optionally, vent 175 (or vents 175) are located lower than the connection to tube 190. This enables injection head 128 to clear the connection to tube 190 without causing liquid to drip, since air is released from vent 175.

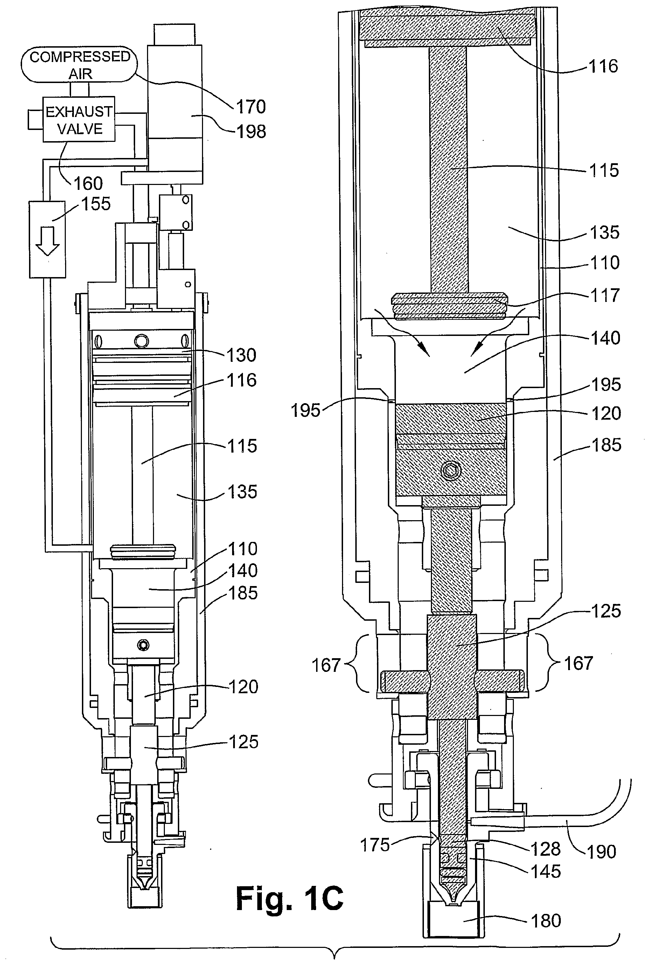

In an exemplary embodiment of the invention, the user of mechanism 100 activates a trigger that causes an exhaust valve 160 to be opened (250) to release the compressed air from chamber 130. This causes piston 115 to move (260) upward and to open the passage for the flow of compressed air from chamber 135 into chamber 140, thus causing the volume of chamber 140 to expand from zero to its maximum size as shown by Fig. 1C relative to Fig. 1B. The compressed air in chamber 140 is thrust upon hammer head 120 causing it to be hurled (270) toward plunger 125 over gap 147 (shown in Fig. 1B). In an exemplary embodiment of the invention, upper plate 116 of piston 115 is larger than lower plate 117 so that the force applied on the bottom plate 116 is greater than that applied on top plate 117 so that piston 115 is forced upward when the trigger is activated. The resulting opening into chamber 140 allows for uninhibited flow of compressed air from chamber 135.

In an exemplary embodiment of the invention, hammer head 120 is hurled (270) toward plunger 125 causing it to jump forward with injection head 128 and slam against the fluid in nozzle 145. This releases an initial amount of fluid at a high pressure that penetrates the epidermis layer and injects some of fluid 165 into the skin of the patient positioned in front of nozzle 145. Optionally a distancing base 180 is positioned in front of nozzle 145 to keep it at a preselected distance from the patient's skin when injecting fluid 165.

In an exemplary embodiment of the invention, hammer head 120 recoils slightly from the collision and then continues forward pushing (280) plunger 125 forward until all of fluid 165 is emptied from nozzle 145 as shown in Fig. 1C.

In some embodiments of the invention, the air left in chamber 140 is released through pores 195, which may be automatically open based on the position of hammer head 120 or controlled by injection mechanism 100. Afterwards the above process may be repeated to enable a continuous series of injections into the skin of the patient.

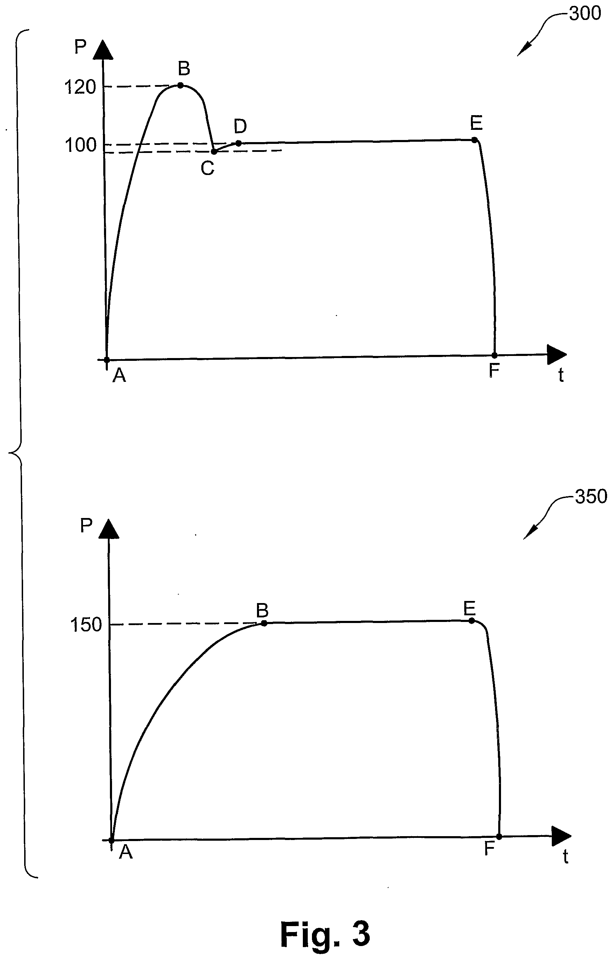

Fig. 3 is an illustration of schematic graphs (300, 350) depicting pressure versus time for a needle-less injection mechanism with a hammer head (300) relative to a needle-less injection mechanism without a hammer head (350), according to an exemplary embodiment of the invention. In an exemplary embodiment of the invention, at point A of graph 300, compressed air 170 is released into chamber 140 causing hammer head 120 to be hurled toward plunger 125. At point B plunger 125 collides with plunger 125 causing plunger 125 to leap forward and release an initial amount of fluid at a maximal pressure (e.g. 120-130 Atmospheres). Hammer head 120 then recoils causing the pressure on fluid 165 to be reduced as shown by point C. The compressed air continues to push against hammer head 120 (point D), pushing it against plunger 125 and causing the rest of the fluid to be injected at an essentially constant pressure (e.g. 100 Atmospheres). In graph 300 the pressure rises a bit from point C to point D and stays essentially steady until point E wherein hammer head 120 and plunger 125 are physically blocked and cannot be pushed any further. At that point all fluid 165 was ejected from nozzle 145. Optionally, hammer head 120 reaches a position where all the compressed air behind it is released (e.g. through pores 195 on encasement 110 at that position). The pressure then drops down at point F as all the compressed air behind hammer head 120 is released.

In an exemplary embodiment of the invention, the pressure applied by hammer head 120 is controlled by adjusting the pressure of the compressed air. Alternatively or additionally, the pressure applied by hammer head 120 is controlled by the impact parameters, for example the distance hammer head 120 needs to travel and it's mass.

In graph 350 a hammer is not used. Optionally, the pressure applied from the time fluid 165 is initially injected (point B) until it finishes being injected (point E) remains essentially constant. In this method there is no initial surge that penetrates the skin to allow the rest of the fluid to follow at a lower pressure. Therefore the entire injection process is conducted at a high pressure.

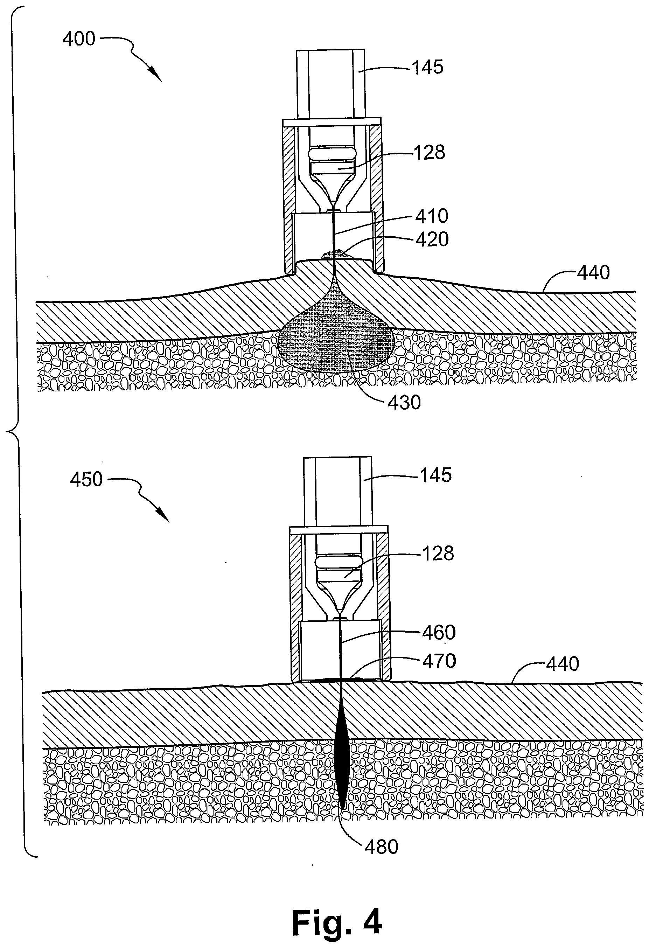

Fig. 4 is an illustration of the dispersion 400 of the injected fluid into the skin using a needle-less injection device with a hammer head 120, relative to the dispersion 450 with a mechanism without a hammer head, according to an exemplary embodiment of the invention. In an exemplary embodiment of the invention, a mushroom shaped dispersion intradermally as shown by 430 in illustration 400 of Fig. 4 will be produced when nozzle 145 is positioned at an optimal position and the compressed air pressure is optimal. An example of optimal values is when nozzle 145 is distanced by 1-6mm from the skin and mechanism 100 with hammer head 120 is calibrated to inject fluid 165 using compressed air so that the initial injection pressure will be between 120-140 Atmospheres and the rest of the fluid will be injected at about 100 Atmospheres.

Optionally in illustration 400 the injected fluid 165 is shown in 3 states:
1) 410 - flowing from nozzle 145 toward epidermis 440.
2) 420 - residual fluid that did not penetrate epidermis 440.
3) 430 - fluid dispersed intradermally or subcutaneously.

In an exemplary embodiment of the invention, when using hammer head 120 in injection mechanism 100, the initial fluid provided at a higher pressure penetrates epidermis 440. The fluid that follows does not need to penetrate epidermis 440 since an entry hole is already formed. The pressure on the fluid that follows is lower so that it does not enter deep below the dermal layer but instead disperses intradermally, forming for example the mushroom shape in dispersion 400. Optionally, if the distance set by base 180 is too far or the pressure is too low more or all of the fluid will remain outside of epidermis 440 as fluid residue 420. On the other hand if the distance set by base 180 is too close or the pressure is too high the fluid will disperse intradermally forming more of a column and less of a mushroom shaped dispersion. This provides us with the ability to control the intradermal dispersion based on the distance of the nozzle from the epidermis and based on the pressure applied to inject the fluid. This ability is especially important for applications wherein we are interested in covering wide covering wide areas close to the epidermis, for example aesthetic applications.

In dispersion 450 fluid 165 is shown flowing 460 from nozzle 145 to epidermis 440. If the pressure is high enough (e.g. 120-150 Atmospheres), fluid 165 will penetrate the epidermis 440 and disperse forming a column 480 due to the high pressure. Optionally, some of the fluid 165 will remain as residual fluid 470 without passing through the epidermis. A greater distance between the nozzle to the epidermis and the lower the pressure will result in more residual fluid 470 and less fluid entering the epidermis. Conversely more fluid will enter epidermis 440 and the column 480 will be narrower and deeper based on:
1. The distance between nozzle 145 and epidermis 440; and
2. The magnitude of the pressure applied to inject fluid 165.
Since the pressure is generally uniform in dispersion 450 there is less control of the spreading out of the dispersion.

It should be appreciated that the above described methods and apparatus may be varied in many ways, including omitting or adding steps, changing the order of steps and the type of devices used, for example the mechanism may be based mainly on electromagnetic mechanisms instead of using compressed air. It should be appreciated that different features may be combined in different ways. In particular, not all the features shown above in a particular embodiment are necessary in every embodiment of the invention. Further combinations of the above features are also considered to be within the scope of some embodiments of the invention.

It will be appreciated by persons skilled in the art that the present invention is not limited to what has been particularly shown and described hereinabove. Rather the scope of the present invention is defined only by the claims, which follow.

## Claims

1. A needle-less fluid injection mechanism (100), comprising:
a nozzle head (145) for accepting a dose of fluid (165) to be injected into the skin of a patient;
a plunger (125) that is adapted to be pushed into the nozzle (145) to inject the dose of fluid (165) from the nozzle (145);
a hammer (120) that is adapted to be thrust at and to collide with said plunger (125) to cause the release of a first amount of fluid (165) from the nozzle (145) at a high pressure and then to release the rest of the fluid (165) from the nozzle (145) by continuing to push said plunger (125) at a lower pressure until injecting the entire dose of fluid (165);
**characterized in that** the needle-less fluid injection mechanism (100) further comprises a position motor (198) to position said plunger (125) before thrusting said hammer (120).

2. A needle-less fluid injection mechanism (100) according to claim 1, further comprising a two plated piston (115) which separates between 3 air chambers (130, 135, 140).

3. A needle-less fluid injection mechanism (100) according to claim 2, wherein the release of air pressure (170) from the uppermost chamber (130) causes the two plated piston (115) to move and release the air pressure (170) from the middle chamber (135) toward the hammer (120).

4. A needle-less fluid injection mechanism (100) according to claim 1, wherein said positioning motor (198) is adapted to form a gap (147) between said hammer (120) and said plunger (125) before thrusting said hammer (120) toward said plunger (125).

5. A needle-less fluid injection mechanism (100) according to claim 1, wherein the level of the pressure by which the first amount of fluid (165) is released is controlled by adjusting the pressure of the compressed air (170) used.

6. A needle-less fluid injection mechanism (100) according to claim 1, wherein the level of the pressure by which the first amount of fluid (165) is released is controlled by adjusting the impact parameters of the hammer (120).

7. A needle-less fluid injection mechanism (100) according to claim 1, wherein compressed air (170) is used to thrust said hammer (120) toward said plunger (125) and to continue pushing said plunger (125) with said hammer (120).

## Patentansprüche

1. Nadelloser Flüssigkeits-Injektionsmechanismus (100), umfassend:
einen Düsenkopf (145) zur Aufnahme einer Dosis der Flüssigkeit (165), die in die Haut eines Patienten injiziert werden soll,
einen Kolben (125), dafür ausgelegt, in die Düse (145) geschoben zu werden, um die Dosis der Flüssigkeit (165) aus der Düse (145) zu injizieren,
einen Hammer (120), dafür ausgelegt, auf den genannten Kolben (125) gestoßen zu werden und auf ihn zu treffen, um die Abgabe einer ersten Menge an Flüssigkeit (165) aus der Düse (145) unter hohem Druck zu bewirken und danach durch fortgesetztes Schieben des genannten Kolbens (125) den Rest der Flüssigkeit (165) unter niedrigerem Druck aus der Düse (145) abzugeben, bis die gesamte Dosis der Flüssigkeit (165) injiziert ist,
**dadurch gekennzeichnet, dass** der nadellose Flüssigkeits-Injektionsmechanismus (100) außerdem einen Stellmotor (198) umfasst, um den genannten Kolben (125) vor dem Stoß des genannten Hammers (120) in Stellung zu bringen.

2. Nadelloser Flüssigkeits-Injektionsmechanismus (100) nach Patentanspruch 1, außerdem einen Zweiplattenkolben (115) umfassend, der 3 Luftkammern (130, 135, 140) voneinander trennt.

3. Nadelloser Flüssigkeits-Injektionsmechanismus (100) nach Patentanspruch 2, in dem die Abgabe von Luftdruck (170) aus der obersten Kammer (130) den Zweiplattenkolben (115) veranlasst, sich zu bewegen und den Luftdruck (170) aus der mittleren Kammer (135) an den Hammer (120) abzugeben.

4. Nadelloser Flüssigkeits-Injektionsmechanismus (100) nach Patentanspruch 1, in dem der genannte Stellmotor (198) dafür ausgelegt ist, eine Lücke (147) zwischen dem genannten Hammer (120) und dem genannten Kolben (125) zu erzeugen, bevor der genannte Hammer (120) gegen den genannten Kolben (125) gestoßen wird.

5. Nadelloser Flüssigkeits-Injektionsmechanismus (100) nach Patentanspruch 1, in dem das Druckniveau, durch das die erste Menge an Flüssigkeit (165) abgegeben wird, durch Einstellung des angewandten Druckes der Druckluft (170) reguliert wird.

6. Nadelloser Flüssigkeits-Injektionsmechanismus (100) nach Patentanspruch 1, in dem das Druckniveau, durch das die erste Menge an Flüssigkeit (165) abgegeben wird, durch Festlegung der Auftreffparameter des Hammers (120) reguliert wird.

7. Nadelloser Flüssigkeits-Injektionsmechanismus (100) nach Patentanspruch 1, in dem Druckluft (170) verwendet wird, um den genannten Hammer (120) gegen den genannten Kolben (125) zu stoßen und den genannten Kolben (125) mit dem genannten Hammer (120) weiterzuschieben.

## Revendications

1. Mécanisme d'injection de liquide sans aiguille (100), comprenant :
- une tête de buse (145) pour recevoir une dose de liquide (165) pour une injection intradermique d'un patient,
- un plongeur (125) pour être poussé dans la buse (145) et injecter la dose de liquide (165) de la buse (145),
- un marteau (120) pour être poussé vers le plongeur (125) et le frapper pour libérer une première quantité de liquide (165) par la buse (145) à haute pression puis libérer le restant de liquide (165) de la buse (145) en continuant à pousser le plongeur (125) à une pression inférieure jusqu'à l'injection de l'ensemble de la dose de liquide (165),
mécanisme d'injection de liquide sans aiguille (100),
**caractérisé en ce qu'**
il comporte en outre un moteur de positionnement (198) pour positionner le plongeur (125) avant de pousser le marteau (120).

2. Mécanisme d'injection de liquide sans aiguille (100) selon la revendication 1,
comportant en outre un piston à deux plateaux (115) qui séparent environ trois chambres à air (130, 135, 140).

3. Mécanisme d'injection de liquide sans aiguille (100) selon la revendication 2,
selon lequel la libération de la pression d'air (170) de la chambre supérieure (130) déplace le piston à deux plateaux (115) pour libérer la pression d'air (170) de la chambre médiane (135) vers le marteau (120).

4. Mécanisme d'injection de liquide sans aiguille (100) selon la revendication 1,
selon lequel le moteur de positionnement (198) forme un intervalle (147) entre le marteau (120) et le plongeur (125) avant de pousser le marteau (120) vers le plongeur (125).

5. Mécanisme d'injection de liquide sans aiguille (100) selon la revendication 1,
dans lequel le niveau de la pression à laquelle la première quantité de liquide (165) est libérée, est commandé par le réglage de la pression de l'air comprimé (170).

6. Mécanisme d'injection de liquide sans aiguille (100) selon la revendication 1,
selon lequel le niveau de la pression à laquelle la première quantité de liquide (165) est libérée, est contrôlé par le réglage des paramètres de l'impact du marteau (120).

7. Mécanisme d'injection de liquide sans aiguille (100) selon la revendication 1,
dans lequel l'air comprimé (170) est utilisé pour pousser le marteau (120) vers le plongeur (125) et continuer à pousser le plongeur (125) avec le marteau (120).
